# EUROPÄISCHE PATENTSCHRIFT

(11) **EP 0 591 799 B1**
(45) Veröffentlichungstag und Bekanntmachung des Hinweises auf die Patenterteilung: **15.01.1997**
(21) Anmeldenummer: 93115540.2
(22) Anmeldetag: 27.09.1993
(51) Int. Cl.: C07C 37/56, C07C 39/10

(54) **Oxidation von Hydroxybenzaldehyden zur Herstellung von Dihydroxybenzol Verbindungen**
Oxidation of hydroxybenzaldehydes to dihydroxybenzene compounds
Oxydation d'hydroxybenzaldéhydes pour l'obtention de dihydroxybenzènes

(30) Priorität: 09.10.1992 DE 4233989
(43) Veröffentlichungstag der Anmeldung: 13.04.1994
(73) Patentinhaber: HOECHST AKTIENGESELLSCHAFT, 65929 Frankfurt am Main (DE)
(72) Erfinder: Weisse, Laurent, Dr., D-61440 Oberursel (DE); Strutz, Heinz, Dr., D-61250 Usingen (DE)

(56) Entgegenhaltungen:
- US-A- 5 120 884
- K F WEDEMEYER 'Methoden Der Organischen Chemie (Houben-Weyl) Band VI/1c Phenole Teil 1' 1976 , GEORG THIEME VERLAG , STUTTGART,DE * Seite 286 - Seite 308 *

## Beschreibung

Die vorliegende Erfindung betrifft ein Verfahren zur Herstellung aromatischer Polyhydroxyverbindungen durch Oxidation der entsprechenden aromatischen Hydroxyaldehyde.

Aromatische Polyhydroxy- insbesondere aromatische Dihydroxyverbindungen besitzen bei der Herstellung von Farbstoffen, in der Kunststoffproduktion, bei der Herstellung wichtiger Pflanzenschutzmittel oder in der Photographie technische Bedeutung (DOS 24 47 971; EP 69 070; DPS 3 308 769).

Zur Herstellung dieser technisch relevanten Verbindungsklasse ist eine Vielzahl von Methoden bekannt, die jedoch meist unselektiv verlaufen (Advanced Organic Chemistry, 1983, McGraw-Hill, Tokyo, 496).

In der EP 436 410 ist eine selektive Synthese substituierter Hydrochinone beschrieben. Danach wird o-Kresol zunächst in para-Stellung regioselektiv acetyliert und das Produkt anschließend mittels der Dakin-Oxidation in das Methylhydrochinon überführt. Nachteil des Verfahrens ist, neben dem Einsatz von teurem Acetylchlorid, der auf den Einsatzstoff bezogene stöchiometrische Verbrauch einer Base.

Auch die Dakin-Oxidation von Hydroxybenzaldehyden benötigt nach Lehre der US 3 585 243 einen Zusatz von mindestens einem halben Äquivalent Base je Mol Hydroxybenzaldehyd.

Die Baeyer-Villiger-Oxidation von Hydroxybenzaldehyden (Houben-Weyl, Methoden der Organischen Chemie, Band VI 1c, 1976; Synthesis (1989) 167) besitzt gegenüber der Dakin-Oxidation den Vorteil, als Koppelprodukte meist nur flüchtige Säuren zu bilden. Die entsprechenden Persäuren, wie Perameisensäure oder Peressigsäure, sind aber sicherheitstechnisch nicht unbedenklich und führen oft zu den Säureestern der entsprechenden Polyhydroxyaromaten, die als Neben- oder Hauptprodukte anschließend aufwendig getrennt oder in einem zusätzlichen Schritt hydrolysiert werden müssen.

Eine Variante dieser Methode ist die durch Säure katalysierte Baeyer-Villiger-Oxidation unter Verwendung von Wasserstoffperoxid als Oxydans und Methanol als Lösungsmittel. Bei diesem Verfahren werden je Mol Einsatzstoff allerdings 15 bis 75 Mol.-% einer Säure, die nach Abschluß der Reaktion neutralisiert werden muß, benötigt (J. Org. Chem. 49 (1984) 4740). Zudem werden bei diesem Verfahren nur die geschützten Phenole, nämlich in Form ihrer Methylether, als Ausgangssubstanzen eingesetzt.

Es besteht daher ein Bedarf an einem Verfahren, das die Nachteile der vorstehend beschriebenen Arbeitsweisen vermeidet. Das Verfahren soll sich zudem auf einfache Weise ausführen lassen und sich technisch leicht zugänglicher Hilfsstoffe bedienen. Es soll darüber hinaus das gewünschte Wertprodukt ohne großen technischen Aufwand in hoher Ausbeute und hoher Reinheit liefern.

Gelöst wird diese Aufgabe durch ein Verfahren zur Herstellung aromatischer Polyhydroxyverbindungen. Es ist dadurch gekennzeichnet, daß man einen aromatischen Hydroxyaldehyd in Anwesenheit eines Nitrils R-CN, worin R für einen Alkylrest mit 1 bis 4 Kohlenstoffatomen oder einen gegebenenfalls durch eine oder mehrere Alkylgruppen substituierten Phenylrest steht, mit Wasserstoffperoxid, gegebenenfalls unter Zusatz einer Base, umsetzt.

Als Einsatzstoff setzt man üblicherweise einen aromatischen Hydroxyaldehyd der allgemeinen Formel (I) 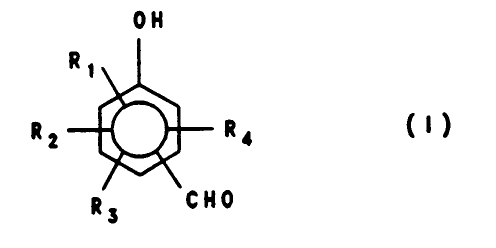 worin R₁, R₂, R₃ und R₄ gleich oder verschieden sind und für Wasserstoff, einen Alkylrest mit 1 bis 20 C-Atomen, einen Arylrest mit 6 bis 14 C-Atomen, einen Alkoxyrest mit 1 bis 10 C-Atomen, einen Arylalkylrest mit 7 bis 20 C-Atomen, einen Alkylarylrest mit 7 bis 20 C-Atomen, einen Aryloxyrest mit 6 bis 10 C-Atomen, einen Fluoralkylrest mit 1 bis 10 C-Atomen, einen Halogenarylrest mit 6 bis 10 C-Atomen, ein Halogenatom oder einen Hydroxylrest stehen oder R₁, R₂, R₃ und/oder R₄ zusammen mit den sie jeweils verbindenden C-Atomen des aromatischen Kerns einen oder mehrere substituierte oder unsubstituierte Ringe bilden, ein.

Zweckmäßig ist es, einen aromatischen Hydroxyaldehyd der allgemeinen Formel (I), worin R₁, R₂, R₃ und R₄ gleich oder verschieden sind und für Wasserstoff, einen Alkylrest mit 1 bis 10 C-Atomen, einen Alkoxyrest mit 1 bis 4 C-Atomen, einen Aryloxyrest mit 6 bis 10 C-Atomen, einen Fluoralkylrest mit 1 bis 6 C-Atomen, ein Halogenatom oder einen Hydroxylrest stehen oder R₁ und R₂, R² und R³ und/oder R³ und R⁴ zusammen mit den sie jeweils verbindenden C-Atomen des aromatischen Kerns einen substituierten oder unsubstituierten, gesättigten oder ungesättigten Ring mit 5 oder 6 Atomen bilden, einzusetzen.

Mit gutem Erfolg kann man in das erfindungsgemäße Verfahren einen aromatischen Hydroxyaldehyd der allgemeinen Formel (I), worin R₁, R₂, R₃ und R₄ gleich oder verschieden sind und für einen Alkylrest mit 1 bis 4 C-Atomen, einen Alkoxyrest mit 1 bis 4 C-Atomen, ein Halogen, insbesondere Fluor oder Chlor, oder einen Hydroxylrest stehen oder R₁ und R₂, R₂ und R₃ oder R₃ und R₄ zusammen mit den sie jeweils verbindenden C-Atomen des aromatischen Kerns einen ungesättigten Ring mit 6 C-Atomen bilden, einsetzen.

Besonders geeignet als Einsatzstoff sind aromatische Hydroxyaldehyde der allgemeinen Formel (I), die einen einzigen Hydroxylrest aufweisen und der Hydroxylrest und die Aldehydgruppe zueinander in 1,2- oder 1,4-Stellung stehen. Namentliche Vertreter dieser Gruppe sind 2-Hydroxybenzaldehyd und 4-Hydroxybenzaldehyd.

In der Regel setzt man nur einen aromatischen Hydroxyaldehyd in die Reaktion ein. Es ist jedoch auch möglich, Gemische zweier oder mehrerer aromatischer Hydroxyaldehyde als Einsatzstoff zu verwenden.

Zur Durchführung des Verfahrens führt man den aromatischen Hydroxyaldehyd, das Nitril R-CN und Wasserstoffperoxid, gegebenenfalls unter Zusatz einer Base und/oder eines Lösungsmittels, zusammen und läßt unter Durchmischung reagieren. Auf den Einsatz der Base kann erforderlichenfalls verzichtet werden. Allerdings führt ein Zusatz der Base zu einer Beschleunigung der Umsetzung. Falls gewünscht, kann man ein Lösungsmittel, beispielsweise einen aliphatischen Alkohol mit 1 bis 4 Kohlenstoffatomen, Essigsäuremethylester, Essigsäureethylester oder Mischungen derselben, verwenden. Man kann aber auch, ohne Nachteile in Kauf nehmen zu müssen, auf das Lösungsmittel erforderlichenfalls verzichten. Bei Zugabe der einzelnen Stoffe ist man an keine besondere Reihenfolge gebunden. Man kann die einzelnen Stoffe gleichzeitig zudosieren oder sie nacheinander zusetzen und das Verfahren kontinuierlich oder diskontinuierlich ablaufen lassen.

Besonders einfach ist es, den aromatischen Hydroxyaldehyd und das Nitril oder ein Gemisch beider Stoffe vorzulegen und das Wasserstoffperoxid oder ein Gemisch von Wasserstoffperoxid und Nitril anschließend zudosieren.

Das molare Verhältnis aromatischer Hydroxyaldehyd:Nitril kann in weiten Grenzen gewählt werden. Es beträgt zweckmäßigerweise 1:(1 bis 50), insbesondere 1:(5 bis 40), bevorzugt 1:(10 bis 30). Überschüssig verwendetes Nitril dient gegebenenfalls als Lösungsmittel.

Man setzt den aromatischen Hydroxyaldehyd in Anwesenheit eines Nitrils R-CN, worin R für einen Alkylrest mit 1 bis 4, insbesondere 1 bis 2 Kohlenstoffatomen oder einen gegebenenfalls durch eine oder mehrere Alkylgruppen, insbesondere Alkylgruppen mit 1 bis 4 Kohlenstoffatomen substituierten Phenylrest, insbesondere einen unsubstituierten Phenylrest steht, um. Es lassen sich auch Mischungen zweier oder mehrerer der vorstehend genannten Nitrile verwenden. Gut geeignete Nitrile sind Acetonitril und Benzonitril, insbesondere Acetonitril.

Die für die Umsetzung benötigte Menge Wasserstoffperoxid richtet sich nach den Aldehydäquivalenten, die zu oxidieren sind. Üblicherweise setzt man je Äquivalent aromatischen Hydroxyaldehyd bzw. je Mol aromatischen Hydroxyaldehyd 0,5 bis 5, insbesondere 0,8 bis 2, bevorzugt 0,9 bis 1,5 Mol Wasserstoffperoxid ein. In den meisten Fällen wird man aromatischen Hydroxyaldehyd und Wasserstoffperoxid im Verhältnis 1:(1 bis 1,2) einsetzen.

Wasserstoffperoxid wird in Form seiner wäßrigen Lösungen mit einem Gehalt von 3 bis 90, insbesondere 5 bis 60, bevorzugt 10 bis 30 Gew.-% H₂O₂ verwendet. Anstelle von Wasserstoffperoxid können auch Wasserstoffperoxid bildende Substanzen oder Gemische genutzt werden. Die Verwendung von wäßrigem Wasserstoffperoxid erlaubt ein besonders einfaches Arbeiten.

Wie zuvor erwähnt, empfiehlt es sich, zur Beschleunigung der Umsetzung eine Base in untergeordneter Menge zu verwenden. Üblicherweise setzt man je Äquivalent aromatischen Hydroxyaldehyd bzw. je Mol aromatischen Hydroxyaldehyd 0,001 bis 0,05 Mol Base zu. Als Base kommen Alkalimetall-und Erdalkalimetallhydroxide und/oder Alkalimetallcarbonate, insbesondere Natriumhydroxid, Kaliumhydroxid und/oder Natriumcarbonat in Betracht.

Man führt die Reaktion bei -30 bis 100°C, insbesondere -10 bis 80°C, bevorzugt 20 bis 70°C durch, wobei eine gute Durchmischung der Reaktionsteilnehmer sicherzustellen ist.

Nach Beendigung der Reaktion wird eventuell noch vorhandenes Wasserstoffperoxid bzw. restliches Peroxid, das sich während der Umsetzung gebildet haben kann, nach bekannten Methoden, beispielsweise durch Zusatz u.a. von Aktivkohle, Pd auf Aktivkohle, Natriumsulfit zerstört, das Nitril weitgehend, gegebenenfalls unter reduziertem Druck, abdestilliert. Anschließend wird der Rückstand in einem geeigneten Lösungsmittel, beispielsweise Essigester, aufgenommen, mit Wasser oder einer gesättigten wäßrigen Salzlösung, insbesondere einer gesättigten wäßrigen NaCl-Lösung versetzt, die organische Phase abgetrennt und die wäßrige Phase mit dem gleichen Lösungsmittel mehrfach extrahiert. Das sich in der wäßrigen Phase befindliche Amid, insbesondere Acetamid, kann als Wertprodukt gewonnen werden oder durch bekannte Verfahren (Beyer, Lehrbuch der organischen Chemie, Hirzel Verlag Stuttgart, 1981, S. 252) in das Nitril zurückgeführt werden. Die organischen Phasen werden vereinigt, getrocknet und durch Destillation vom Lösungsmittel befreit. Der dabei anfallende Rückstand enthält das Wertprodukt bereits in hoher Reinheit (≥95 %).

Das Verfahren läßt sich besonders einfach unter Atmosphärendruck ausführen. Falls gewünscht, ist es auch möglich, unterhalb oder oberhalb des Atmosphärendrucks zu arbeiten.

Das erfindungsgemäße Verfahren zeichnet sich nicht nur durch einen nahezu quantitativen Umsatz des aromatischen Hydroxyaldehyds, sondern auch durch eine ungewöhnlich hohe Selektivität hinsichtlich der Bildung der gewünschten Polyhydroxyverbindungen aus. Nebenprodukte, wie Hydroxycarbonsäuren und Hydroxycarbonsäureester werden nicht gebildet. Daher kann üblicherweise auf eine aufwendige Reinigung des anfallenden Reaktionsproduktes verzichtet werden.

Da die Base lediglich in geringem Umfange (katalytische Mengen) zugesetzt wird, fallen umweltbelastende Salze und mit Salzen beladene Abwässer ebenfalls nur in sehr niedrigen Mengen an. Demzufolge erweist sich das erfindungsgemäße Verfahren gegenüber den Verfahren des Standes der Technik, die in wesentlich höherem Maße zu Salzen führen, als umweltfreundlicher.

Die nachfolgenden Beispiele belegen die Erfindung.

### Experimenteller Teil

### Beispiel 1

### Herstellung von Brenzcatechin (o-Dihydroxybenzol)

6,1 g (0,05 mol) Salicylaldehyd und 0,01 g (0,25 mmol) NaOH in 50 ml (0,96 mol) Acetonitril werden vorgelegt und mit 17,2 g einer 11,8 %igen (0,06 mol) wäßrigen Wasserstoffperoxidlösung versetzt und 48 Stunden bei 50°C gerührt.
Eventuell noch vorhandene Peroxide werden mit einer verdünnten Natriumsulfitlösung entfernt. Das Reaktionsgemisch wird anschließend mit Essigester versetzt, die organische Phase abgetrennt und wäßrige Phase mehrmals mit Essigester gewaschen. Anschließend werden die vereinigten und getrockneten organischen Phasen im Vakuum vom Lösungsmittel befreit, wobei 5,4 g eines Feststoffs erhalten werden, der anhand einer Vergleichsprobe ¹H-NMR-spektroskopisch als Brenzcatechin identifiziert wird.
(Reinheit ermittelt durch Gaschromatographie: 98 %; Ausbeute 96 % der Theorie).

### Beispiel 2

### Herstellung von Hydrochinon (p-Dihydroxybenzol)

6,1 g (0,05 mol) p-Hydroxybenzaldehyd und 0,05 g (1,25 mmol) NaOH werden analog Beispiel 1 24 Stunden umgesetzt und aufgearbeitet. Man erhält 5,35 g eines Feststoffes, der anhand einer Vergleichsprobe ¹H-NMR-spektroskopisch als Hydrochinon identifiziert wird.
(Reinheit ermittelt durch Gaschromatographie 98 %; Ausbeute: 95 % der Theorie).

### Beispiel 3

### Herstellung von 2,3-Dimethylhydrochinon

15 g (0,1 mol) 3,5-Dimethyl-4-hydroxybenzaldehyd, 0,1 g (2,5 mmol) NaOH und 34,4 g einer 11,8 %igen (0,12 mol) wäßrigen Wasserstoffperoxidlösung werden analog Beispiel 1 umgesetzt und aufgearbeitet. Man erhält 13,7 g eines Feststoffes, der anhand einer Vergleichsprobe ¹H-NMR-spektroskopisch als 2,3-dimethylhydrochinon identifiziert wird.
(Reinheit ermittelt durch Gaschromatographie: 98 %; Ausbeute 97 % der Theorie).

### Beispiel 4

### Herstellung von Brenzcatechin

6,1 g (0,05 mol) Salicylaldehyd und 33 g einer 12,6 %igen (0,13 mol) wäßrigen Wasserstoffperoxidlösung werden analog Beispiel 1, jedoch ohne Basenzusatz 70 Stunden bei 50°C umgesetzt und aufgearbeitet. Man erhält 5,4 g eines Feststoffs, der anhand einer Vergleichsprobe ¹H-NMR-spektroskopisch als Brenzcatechin identifiziert wird.
(Reinheit ermittelt durch Gaschromatographie: 90 %; Ausbeute 88 % der Theorie).

## Patentansprüche

1. Verfahren zur Herstellung aromatischer Polyhydroxyverbindungen, dadurch gekennzeichnet, daß man einen aromatischen Hydroxyaldehyd in Anwesenheit eines Nitrils R-CN, worin R für einen Alkylrest mit 1 bis 4 Kohlenstoffatomen oder einen gegebenenfalls durch eine oder mehrere Alkylgruppen substituierten Phenylrest steht, mit Wasserstoffperoxid, gegebenenfalls unter Zusatz einer Base, umsetzt.

2. Verfahren nach Anspruch 1, dadurch gekennzeichnet, daß man einen aromatischen Hydroxyaldehyd der allgemeinen Formel (I) 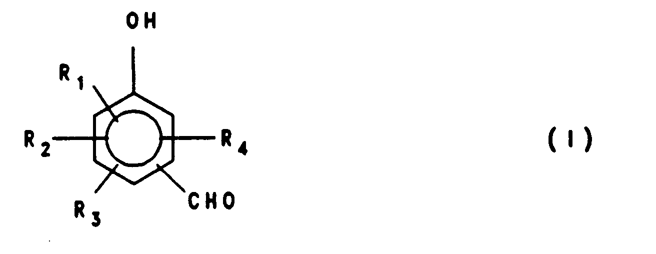 worin R₁, R₂, R₃ und R₄ gleich oder verschieden sind und für Wasserstoff, einen Alkylrest mit 1 bis 20 C-Atomen, einen Arylrest mit 6 bis 14 C-Atomen, einen Alkoxyrest mit 1 bis 10 C-Atomen, einen Arylalkylrest mit 7 bis 20 C-Atomen, einen Alkylarylrest mit 7 bis 20 C-Atomen, einen Aryloxyrest mit 6 bis 10 C-Atomen, einen Fluoralkylrest mit 1 bis 10 C-Atomen, einen Halogenarylrest mit 6 bis 10 C-Atomen, ein Halogenatom oder einen Hydroxylrest stehen oder R₁, R₂, R₃ und/oder R₄ zusammen mit den sie jeweils verbindenden C-Atomen des aromatischen Kerns einen oder mehrere substituierte oder unsubstituierte Ringe bilden, einsetzt.

3. Verfahren nach Anspruch 1 oder 2, dadurch gekennzeichnet, daß man einen aromatischen Hydroxyaldehyd der allgemeinen Formel (I), worin R₁, R₂, R₃ und R₄ gleich oder verschieden sind und für Wasserstoff, einen Alkylrest mit 1 bis 10 C-Atomen, einen Alkoxyrest mit 1 bis 4 C-Atomen, einen Aryloxyrest mit 6 bis 10 C-Atomen, einen Fluoralkylrest mit 1 bis 6 C-Atomen, ein Halogenatom oder einen Hydroxylrest stehen oder R¹ und R², R² und R³ und/oder R³ und R⁴ zusammen mit den sie jeweils verbindenden C-Atomen des aromatischen Kerns einen substituierten oder unsubstituierten, gesättigten oder ungesättigten Ring mit 5 oder 6 Atomen bilden, einsetzt.

4. Verfahren nach einem oder mehreren der Ansprüche 1 bis 3, dadurch gekennzeichnet, daß man einen aromatischen Hydroxyaldehyd der allgemeinen Formel (I), worin R₁, R₂, R₃ und R₄ gleich oder verschieden sind und für einen Alkylrest mit 1 bis 4 C-Atomen, einen Alkoxyrest mit 1 bis 4 C-Atomen, ein Halogen, insbesondere Fluor oder Chlor, oder einen Hydroxylrest stehen oder R₁ und R₂, R₂ und R₃ oder R₃ und R₄ zusammen mit den sie jeweils verbindenden C-Atomen des aromatischen Kerns einen ungesättigten Ring mit 6 C-Atomen bilden, einsetzt.

5. Verfahren nach einem oder mehreren der Ansprüche 1 bis 4, dadurch gekennzeichnet, daß der aromatische Hydroxyaldehyd der allgemeinen Formel (I) einen einzigen Hydroxylrest aufweist und der Hydroxylrest und die Aldehydgruppe in 1,2- oder 1,4-Stellung zueinander stehen.

6. Verfahren nach einem oder mehreren der Ansprüche 1 bis 5, dadurch gekennzeichnet, daß man je Mol aromatischen Hydroxyaldehyd 1 bis 50, insbesondere 5 bis 40, bevorzugt 10 bis 30 Mol Nitril einsetzt.

7. Verfahren nach einem oder mehreren der Ansprüche 1 bis 6, dadurch gekennzeichnet, daß man als Nitril Acetonitril und/oder Benzonitril, insbesondere Acetonitril, einsetzt.

8. Verfahren nach einem oder mehreren der Ansprüche 1 bis 7, dadurch gekennzeichnet, daß man je Mol aromatischen Hydroxyaldehyd 0,5 bis 5, insbesondere 0,8 bis 2, bevorzugt 0,9 bis 1,5 Mol Wasserstoffperoxid einsetzt.

9. Verfahren nach einem oder mehreren der Ansprüche 1 bis 8, dadurch gekennzeichnet, daß man je Mol aromatischen Hydroxyaldehyd 0,001 bis 0,05 Mol Base einsetzt.

10. Verfahren nach einem oder mehreren der Ansprüche 1 bis 9, dadurch gekennzeichnet, daß man als Base ein Alkalimetallhydroxid, ein Erdalkalimetallhydroxid und/oder ein Alkalimetallcarbonat einsetzt.

11. Verfahren nach einem oder mehreren der Ansprüche 1 bis 10, dadurch gekennzeichnet, daß man die Umsetzung bei -30 bis 100, insbesondere -10 bis 80 °C durchführt.

## Claims

1. A process for the preparation of aromatic polyhydroxy compounds, which comprises reacting an aromatic hydroxyaldehyde with hydrogen peroxide, with or without the addition of a base, in the presence of a nitrile R-CN, in which R is an alkyl radical having 1 to 4 carbon atoms or a phenyl radical, which is unsubstituted or substituted by one or more alkyl groups.

2. The process as claimed in claim 1, wherein an aromatic hydroxyaldehyde is used of the formula (I) 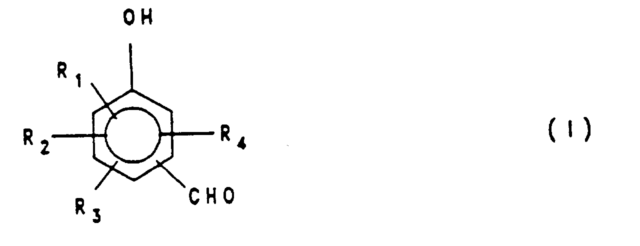 in which R₁, R₂, R₃ and R₄ are identical or different and are hydrogen, an alkyl radical having 1 to 20 carbon atoms, an aryl radical having 6 to 14 carbon atoms, an alkoxy radical having 1 to 10 carbon atoms, an arylalkyl radical having 7 to 20 carbon atoms, an alkylaryl radical having 7 to 20 carbon atoms, an aryloxy radical having 6 to 10 carbon atoms, a fluoroalkyl radical having 1 to 10 carbon atoms, a haloaryl radical having 6 to 10 carbon atoms, a halogen atom or a hydroxyl radical or R₁, R₂, R₃ and/or R₄, together with the respective connecting carbon atoms of the aromatic nucleus, form one or more substituted or unsubstituted rings.

3. The process as claimed in claim 1 or 2, wherein an aromatic hydroxyaldehyde of the formula (I) is used, in which R₁, R₂, R₃ and R₄ are identical or different and are hydrogen, an alkyl radical having 1 to 10 carbon atoms, an alkoxy radical having 1 to 4 carbon atoms, an aryloxy radical having 6 to 10 carbon atoms, a fluoroalkyl radical having 1 to 6 carbon atoms, a halogen atom or a hydroxyl radical or R₁ and R₂, R₂ and R₃ and/or R₃ and R₄, together with the respective connecting carbon atoms of the aromatic nucleus, form a substituted or unsubstituted, saturated or unsaturated ring having 5 or 6 atoms.

4. The process as claimed in one or more of claims 1 to 3, wherein an aromatic hydroxyaldehyde of the formula (I) is used, in which R₁, R₂, R₃ and R₄ are identical or different and are an alkyl radical having 1 to 4 carbon atoms, an alkoxy radical having 1 to 4 carbon atoms, a halogen, in particular fluorine or chlorine, or a hydroxyl radical or R₁ and R₂, R₂ and R₃ or R₃ and R₄, together with the respective connecting carbon atoms of the aromatic nucleus, form an unsaturated ring having 6 carbon atoms.

5. The process as claimed in one or more of claims 1 to 4, wherein the aromatic hydroxyaldehyde of the formula (I) has a single hydroxyl radical and the hydroxyl radical and the aldehyde group are situated in the 1,2- or 1,4-position relative to each other.

6. The process as claimed in one or more of claims 1 to 5, wherein, per mole of aromatic hydroxyaldehyde, 1 to 50, in particular 5 to 40, preferably 10 to 30 mol of nitrile are used.

7. The process as claimed in one or more of claims 1 to 6, wherein the nitrile used is acetonitrile and/or benzonitrile, in particular acetonitrile.

8. The process as claimed in one or more of claims 1 to 7, wherein, per mole of aromatic hydroxyaldehyde, 0.5 to 5, in particular 0.8 to 2, preferably 0.9 to 1.5 mol of hydrogen peroxide are used.

9. The process as claimed in one or more of claims 1 to 8, wherein, per mole of aromatic hydroxyaldehyde, 0.001 to 0.05 mol of base are used.

10. The process as claimed in one or more of claims 1 to 9, wherein the base used is an alkali metal hydroxide, an alkaline earth metal hydroxide and/or an alkali metal carbonate.

11. The process as claimed in one or more of claims 1 to 10, wherein the reaction is carried out at -30 to 100, in particular -10 to 80°C.

## Revendications

1. Procédé de préparation de composés polyhydroxylés aromatiques, caractérisé en ce qu'on fait réagir un hydroxyaldéhyde aromatique avec du peroxyde d'hydrogène, en présence d'un nitrile R-CN, dans lequel R représente un radical alkyle avec 1 à 4 atomes de carbone ou un radical phényle éventuellement substitué par un ou plusieurs groupes alkyle, éventuellement sous l'addition d'une base.

2. Procédé selon la revendication 1, caractérisé en ce qu'on utilise un hydroxyaldéhyde aromatique de formule générale (I) 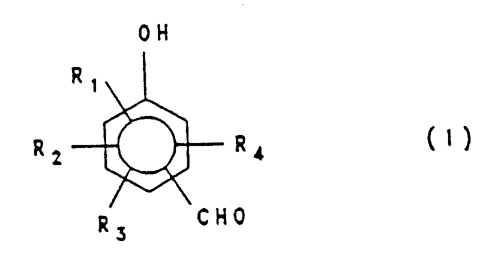 dans laquelle R₁, R₂, R₃ et R₄ sont identiques ou différents et représentent l'hydrogène, un radical alkyle avec 1 à 20 atomes de C, un radical aryle avec 6 à 14 atomes de C, un radical alcoxy avec 1 à 10 atomes de C, un radical aralkyle avec 7 à 20 atomes de C, un radical alkylaryle avec 7 à 20 atomes de C, un radical aryloxy avec 6 à 10 atomes de C, un radical fluoroalkyle avec 1 à 10 atomes de C, un radical halogénoaryle avec 6 à 10 atomes de C, un atome d'halogène ou un radical hydroxyle ou R₁, R₂, R₃ et/ou R₄ forment ensemble, avec les atomes du noyau aromatique qui y sont liés respectivement, un ou plusieurs cycles substitués ou non substitués.

3. Procédé selon la revendication 1 ou 2, caractérisé en ce qu'on utilise un hydroxyaldéhyde aromatique de formule générale (I) dans laquelle R₁, R₂, R₃ et R₄ sont identiques ou différents et représentent l'hydrogène, un radical alkyle avec 1 à 10 atomes de C, un radical alcoxy avec 1 à 4 atomes de C, un radical aryloxy avec 6 à 10 atomes de C, un radical fluoroalkyle avec 1 à 6 atomes de C, un atome d'halogène ou un radical hydroxyle ou R₁ et R₂, R₂ et R₃ et/ou R₃ et R₄ forment, ensemble avec les atomes de C du noyau aromatique auxquels ils sont respectivement liés, un cycle avec 5 ou 6 atomes, saturé ou insaturé, substitué ou non substitué.

4. Procédé selon l'une ou plusieurs des revendications 1 à 3, caractérisé en ce qu'on utilise un hydroxyaldéhyde aromatique de formule générale (I), dans laquelle R₁, R₂, R₃ et R₄ sont identiques ou différents et représentent un radical alkyle avec 1 à 4 atomes de C, un radical alcoxy avec 1 à 4 atomes de C, un halogène, en particulier le fluor ou le chlore, ou un radical hydroxyle, ou R₁ et R₂, R₂ et R₃ ou R₃ et R₄ forment, ensemble avec les atomes de C du noyau aromatique qui y sont respectivement liés, un cycle insaturé avec 6 atomes de C.

5. Procédé selon l'une ou plusieurs des revendications 1 à 4, caractérisé en ce que l'hydroxyaldéhyde aromatique de formule générale (I) présente un radical hydroxyle unique et en ce que le radical hydroxyle et le groupe aldéhyde sont en position 1,2 ou 1,4 l'un par rapport à l'autre.

6. Procédé selon l'une ou plusieurs des revendications 1 à 5, caractérisé en ce qu'on utilise, par mol d'hydroxyaldéhyde aromatique, 1 à 50, en particulier 5 à 40, de préférence 10 à 30 mol de nitrile.

7. Procédé selon l'une ou plusieurs des revendications 1 à 6, caractérisé en ce qu'on utilise comme nitrile de l'acétonitrile et/ou du benzonitrile, en particulier de l'acétonitrile.

8. Procédé selon l'une ou plusieurs des revendications 1 à 7, caractérisé en ce qu'on utilise, par mol d'hydroxyaldéhyde aromatique, 0,5 à 5, en particulier 0,8 à 2, de préférence 0,9 à 1,5 mol de peroxyde d'hydrogène.

9. Procédé selon l'une ou plusieurs des revendications 1 à 8, caractérisé en ce qu'on utilise, par mol d'hydroxyaldéhyde aromatique, 0,001 à 0,05 mol de base.

10. Procédé selon l'une ou plusieurs des revendications 1 à 9, caractérisé en ce qu'on utilise comme base un hydroxyde de métal alcalin, un hydroxyde de métal alcalino-terreux et/ou un carbonate de métal alcalin.

11. Procédé selon l'une ou plusieurs des revendications 1 à 10, caractérisé en ce que la réaction est effectuée dans la gamme de -30 à 100, en particulier de -10 à 80°C.
